# EUROPÄISCHE PATENTANMELDUNG

(11) **EP 0 599 198 A1**
(43) Veröffentlichungstag der Anmeldung: **01.06.1994**
(21) Anmeldenummer: 93118572.2
(22) Anmeldetag: 18.11.1993
(51) Int. Cl.: C07D 233/78, C07D 233/76, C07D 233/74

(54) **Verfahren zur Herstellung von 5-Arylhydantoinen**

(30) Priorität: 27.11.1992 AT 2355/92
(71) Anmelder: Chemie Linz GmbH, A-4021 Linz (AT)
(72) Erfinder: Hendel, Wolfram, Dr., A-4060 Leonding (AT); Kloimstein, Engelbert, Ing., A-4070 Eferding (AT); Fitzinger, Klaus, A-4020 Linz (AT); Viehböck, Antonia, A-4020 Linz (AT); Haidinger, Kurt, A-4040 Linz (AT)
(74) Vertreter: Kunz, Ekkehard, Dr.

(57) **Zusammenfassung**

Verfahren zur Herstellung von 5-Arylhydantoinen durch Umsetzung von Allantoinsäurealkylester mit einer Arylverbindung in konzentrierter anorganischer Säure bei Temperaturen von Raumtemperatur bis 150 °C mit oder ohne einen Phasenvermittler.

## Beschreibung

Arylhydantoine sind wertvolle Zwischenprodukte zur Herstellung enantiomerenreiner Arylaminosäuren, die beispielsweise in der Pharmazie Anwendung finden.
Verfahren zur Herstellung von Arylhydantoinen sind schon seit langem bekannt.
So kann beispielsweise gemäß E.K.Harvill et al., J.Org. Chem. 9, 26 (1944), 5-(4-Hydroxyphenyl)-hydantoin durch Umsetzung von p-Hydroxybenzaldehyd mit Kaliumcyanid und Ammoniumcarbonat in wäßriger,alkoholischer Lösung hergestellt werden. Cyanide sind aber toxisch und werden in technischen Prozessen nur eingesetzt, wenn es unumgänglich ist.
Aus GB-A-2 012 756 geht hervor, daß 5-Arylhydantoine durch Umsetzung von Harnstoff mit einer alpha-Aryl-alpha-Hydroxyessigsäure erhalten werden können. Die alpha-Aryl-alpha-Hydroxysäure wird dabei durch Umsetzung von Glyoxylsäure mit einer Arylverbindung in wässrigem Medium in Gegenwart eines Alkalimetallhydroxides hergestellt, wobei aber schwer zu trennende Isomerengemische entstehen, sodaß diese Reaktion im allgemeinen nur mit geringen Ausbeuten gelingt.
In GB-A-20 53 206 ist die Herstellung von 5-Arylhydantoinen durch Umsetzung von Allantoin mit einem Arylderivat in einem heißen, sauren Medium geoffenbart. Für die Umsetzung muß aber zuerst einmal Allantoin hergestellt werden.
Nach GB-A-1 572 316 kann 5-(4-Hydroxyphenyl)-hydantoin aus einfachen Ausgangsstoffen, nämlich aus Glyoxylsäure, Harnstoff und Phenol in wäßriger, saurer Lösung hergestellt werden. Es hat sich aber herausgestellt, daß das Produkt dieser Reaktion relativ stark durch sein Isomeres 5-(2-Hydroxyphenyl)-hydantoin, das nur schlecht abgetrennt werden kann, verunreinigt ist.
Gemäß EP-A-0 474 112 werden zur Herstellung von 5-Arylhydantoinen mit guter Reinheit Glyoxylsäuremethylestermethylhemiacetal mit Harnstoff und einer Arylverbindung im Eintopfverfahren umgesetzt.
Die langen Reaktionszeiten von 20 Stunden und mehr sind aber für ein technisches Verfahren nicht tragbar.

Unerwarteterweise wurde nun gefunden, daß man 5-Arylhydantoine in einer 2-Stufenreaktion ausgehend von einfachen, ungiftigen Ausgangsstoffen in hoher Reinheit erhält, wenn man in einer 1. Reaktionsstufe ein Glyoxylsäurealkylesteralkylhemiacetal mit Harnstoff zum Allantoinsäurealkylester umsetzt, diesen isoliert und anschließend in einer 2. Reaktionsstufe mit einer Arylverbindung reagieren läßt, wobei die Reaktionszeiten in beiden Stufen erstaunlicherweise sehr kurz sind.

Gegenstand der Erfindung ist daher ein Verfahren zur Herstellung von 5-Arylhydantoinen der allgemeinen Formel 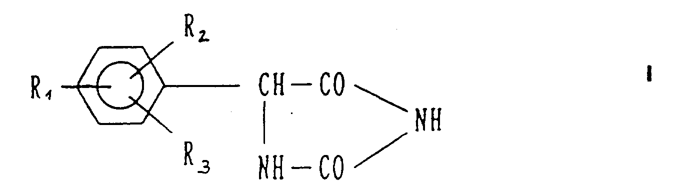
in der R₁, R₂ und R₃ unabhängig voneinander Wasserstoff-, Alkyl- oder Alkoxygruppen mit je 1 bis 6 C-Atomen, Hydroxy-, Halogen- oder durch Acylgruppenn mit 1 bis 8 C-Atomen, durch Alkylgruppen mit 1 bis 6 C-Atomen oder durch Phenylgruppen substituierte Aminogruppen bedeuten, das dadurch gekennzeichnet ist, daß ein Allantoinsäurealkylester der allgemeinen Formel 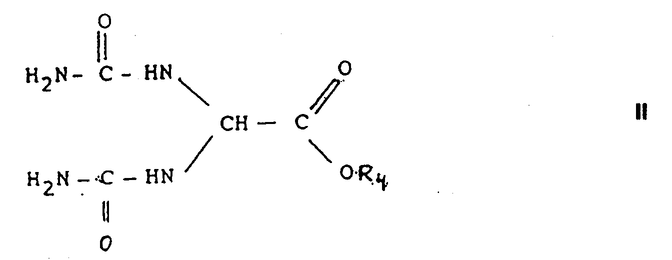
in der R₄ eine Alkylgruppa mit 1 bis 8 C-Atomen bedeutet, in konzentrierter, anorganischer Säure bei Temperaturen von Raumtemperatur bis 150 °C mit oder ohne einen Phasenvermittler mit einer Arylverbindung der allgemeinen Formel 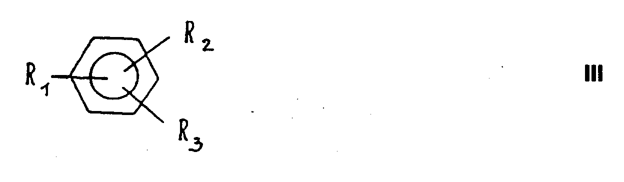
in der R₁, R₂ und R₃ die oben angegebene Bedeutung haben, umgesetzt wird.

In der allgemeinen Formel I bedeuten R₁, R₂ und R₃ unabhängig voneinander Wasserstoff, eine Alkyl-, Alkoxy-, Hydroxy-, Halogen- oder eine durch unter den Reaktionsbedingungen inerte Gruppen substituierte Aminogruppe. Unter Alkyl- oder Alkoxygruppen sind Gruppen mit 1 bis 6, bevorzugt mit 1 bis 3 C-Atomen, beispielsweise Methyl-, Ethyl-, Propyl-, iso-Propyl-, Hexylgruppen, unter Halogen Fluor, Chlor oder Brom zu verstehen. Aminogruppen, die durch unter den Reaktionsbedingungen inerte Gruppen geschützt sind, sind Aminogruppen, die durch Acylgruppen mit 1 bis 8 C-Atomen, bevorzugt durch die Acetylgruppe, durch Alkylgruppen mit 1 bis 6 C-Atomen oder durch Phenylgruppen substituiert sind. Bevorzugt bedeuten R₁, R₂ und R₃ unabhängig voneinander Wasserstoff, Hydroxy-, Alkoxy-, Halogen- oder substituierte Aminogruppen. Ganz besonders bevorzugt bedeuten R₁ Wasserstoff und R₂ und R₃ unabhängig voneinander Wasserstoff, Hydroxy-, Alkoxy- oder durch Alkylgruppen substituierte Aminogruppen.

In der allgemeinen Formel II bedeutet R₄ eine Alkylgruppe mit 1 bis 8 C-Atomen. Unter Alkylgruppe ist dabei eine geradkettige oder verzweigte Alkylgruppe, beispielsweise eine Methyl-, Ethyl-, Propyl-, iso-Propyl, iso-Pentyl-, Hexyl-, Octylgruppe, bevorzugt eine Methyl-, Ethyl-, iso-Propyl-, Butyl- oder Hexylgruppe zu verstehen.
Die Herstellung von Verbindungen der Formel II kann durch Umsetzung eines Glyoxylsäurealkylesteralkylhemiacetals mit Harnstoff in Gegenwart eines Alkylalkohols und einer Mineralsäure erfolgen. Die Herstellung des Allantoinsäuremethylesters aus Glyoxylsäuremethylestermethylhemiacetal und Harnstoff in Gegenwart anorganischer Säure und Methanol ist beispielsweise in EP-A-0 460 412 geoffenbart. Diese Reaktion ist im allgemeinen innerhalb weniger Stunden abgeschlossen.

In der allgemeinen Formel III haben R₁, R₂ und R₃ die in der allgemeinen Formel I angegebene Bedeutung. Ganz besonders bevorzugte Verbindungen der allgemeinen Formel III sind Benzol, Phenol, Dihydroxybenzol, Anisol, Gujacol, Veratrol, Trimethoxybenzol.

Zur Durchführung der erfindungsgemäßen Reaktion werden die Verbindung der Formel II und die Verbindung der Formel III in einer anorganischen Säure vorgelegt.
Im allgemeinen werden pro Mol der Verbindung der Formel II 1 bis 6, bevorzugt 1 bis 3 Mol der Verbindung der Formel III eingesetzt. Es kann aber in Einzelfällen von Vorteil sein, die Verbindung der Formel III im Unterschuß einzusetzen.
Als anorganische Säuren werden Mineralsäuren wie Salzsäure, Schwefelsäure, oder Mischungen solcher Säuren eingesetzt. Die Säuremenge ist abhängig von der Art der Verbindung III, die in manchen Fällen in der Säure nur schwer löslich ist. Um die Löslichkeit der Verbindung III in der Säure zu erhöhen, wird gegebenenfalls ein Phasenvermittler, beispielsweise eine flüssige, organische Säure wie Essigsäure, Propionsäure, bevorzugt Essigsäure eingesetzt.

Die Reaktionsmischung wird bei Temperaturen von etwa Raumtemperatur bis 150 °C, bevorzugt bei Temperaturen von 60 bis 120 °C, besonders bevorzugt von 70 bis 100 °C, gerührt. Gegebenenfalls erfolgt die Reaktion unter Druck, wobei übliche Drücke von 0,3 bis 2MPa angewendet werden.

Dabei bildet sich das 5-Arylhydantoin der Formel I. Der Verlauf der Reaktion kann mit Hilfe üblicher Methoden, beispielsweise Gaschromatographie, Dünnschichtchromatographie, verfolgt werden. Die Reaktion läuft in erstaunlich kurzer Zeit ab und ist in manchen Fällen schon nach 2 Stunden beendet.
Die Position im Arylring der Formel III, in der der Allantoinsäurealkylester der Formel II reagiert, hängt von der Art der Substituenten R₁, R₂ und R₃ und deren jeweiligen Position im Arylring der Formel III ab. Bekanntlich bedingt die Art eines Substituenten in einem Arylring die Ladungsdichteverteilung im Molekül und die Position eines Substituenten im Arylring in weiterer Folge je nach Stärke des induktiven oder mesomeren Effektes jene Position, in der ein weiterer Substituent durch eine Reaktion eingeführt wird. Bei Substituenten im Arylring, die nicht Wasserstoff sind, hängt die Position des Reaktionszentrums im Arylring von der Stärke des jeweiligen induktiven oder mesomeren Effektes der einzelnen Substituenten ab. Es hat sich herausgestellt, daß im Falle, daß in der Formel III R₁ eine Hydroxygruppe und R₂ und R₃ Wasserstoff bedeuten, die Reaktion überwiegend in para-Stellung abläuft. In Einzelfällen, in denen die Position des Reaktionszentrums nicht mit Sicherheit vorhergesagt werden kann, genügt ein Vorversuch, nach dem das hergestellte Produkt charakterisiert werden kann.

Nach beendeter Reaktion wird die Reaktionsmischung abgekühlt. Dabei kann das 5-Arylhydantoin aus der Reaktionsmischung auskristallisieren und durch übliche Methoden wie Filtrieren, Zentrifugieren isoliert werden. Es hat sich unerwarteterweise herausgestellt, daß erfindungsgemäß hergestelltes 5-(4-Hydroxyphenyl)-hydantoin in größeren Kristallen auskristallisiert und daher besonders einfach und gut filtrierbar ist, wobei nach bisher bekannten Herstellungsverfahren 5-(4-Hydroxyphenyl)-hydantoin im allgemeinen sehr feinkristallin anfiel und deshalb Schwierigkeiten bei der Festflüssig Trennung auftraten.
Kristallisiert das 5-Arylhydantoin aus der Reaktionsmischung nicht aus, kann die Reaktionsmischung nach Neutralisieren und gegebenenfalls nach Eindampfen mit Hilfe geeigneter organischer Lösungsmittel extrahiert und das gebildete 5-Arylhydantoin auf diese Weise isoliert werden. Neutralisieren bedeutet Einstellen des pH-Wertes auf einen Wert, bei dem das gebildete 5-Arylhydantoin noch kein Salz bildet.

Üblicherweise wird ein pH-Wert von 3 bis 9 eingestellt. Geeignete organische Lösungsmittel sind etwa Carbonsäureester, z. B. Essigsäureethyl- oder butylester, Ether, z. B. Methy-tert.butylether, Diisopropylether, Tetrahydrofuran, Ketone, z.B. Aceton, Methylethylketon, Diisobutylketon, mit Wasser nicht mischbare Alkohole, chlorierte Kohlenwasserstoffe wie Methylenchlorid, Trichloräthylen. Bevorzugt sind Carbonsäureeester, Ether oder Ketone, besonders bevorzugt Carbonsäureester.

Es hat sich gezeigt, daß die Reinheit des auf die beschriebene Art und Weise isolierten 5-(4-Hydroxyphenyl)-hydantoins im allgemeinen mehr als 99 % beträgt und daher ausreichend ist. Für spezielle Fälle kann die Reinheit des 5-(4-Hydroxyphenyl)-hydantoins durch Erhitzen in Wasser auf Rückfluß noch gesteigert werden. In anderen Fällen kann eine Reinigung durch extraktive oder chromatographische Methoden oder durch Umkristallisieren angeschlossen werden.

In einer bevorzugten Ausführungsform der Erfindung werden pro Mol einer Verbindung der allgemeinen Formel II, in der R₄ eine Alkylgruppe mit 1 bis 6 C-Atomen bedeutet, etwa 2 bis 3 Mol einer Verbindung der Formel III, in der R₁, R₂ und R₃ unabhängig voneinander Wasserstoff, Hydroxy, Alkoxy- oder durch Alkylgruppen substituierte Aminogruppen bedeuten, in konzentrierter Salzsäure oder Schwefelsäure vorgelegt und unter Rühren auf Temperaturen von 70 bis 100 °C erhitzt. Nach beendeter Reaktion wird die Reaktionsmischung abgekühlt. Fällt das Produkt kristallin an, wird abfiltriert, das abfiltrierte Produkt gegebenenfalls mit Wasser auf Rückfluß erhitzt, gekühlt und filtriert.

Fällt das Produkt nicht kristallin an, wird entweder die Reaktionsmischung durch Zugabe von Natronlauge auf einen pH-Wert zwischen 3 und 9, bevorzugt zwischen 4 und 8 gestellt, eingedampft und der Rückstand mit einem Carbonsäureester extrahiert oder der Reaktionsmischung wird Wasser zugegeben und die wäßrige Mischung wird, gegebenenfalls nach Einstellen des pH-Wertes auf pH 3 bis 9 durch Zugabe von Natronlauge, mit einem organischen, mit Wasser nicht mischbaren Lösungsmittel extrahiert. Das organische Lösungsmittel wird gegebenenfalls nach Trocknen abgedampft und der Rückstand gegebenenfalls durch übliche Methoden, wie Umkristallisieren, Chromatographieren noch weiter gereinigt.

Bevorzugt wird in das Verfahren Allantoinsäuremethylester, der durch Umsetzung von Glyoxylsäuremethylestermethylhemiacetal und Harnstoff in Methanol in Gegenwart von Schwefelsäure hergestellt wurde, eingesetzt. Die Herstellung eines 5-Arylhydantoins der allgemeinen Formel I, ausgehend von Glyoxylsäurealkyesteralkylhemiacetal und Harnstoff, die in einer ersten Stufe gemäß der in EP-A-0 460 412 geoffenbarten Art und Weise zum Allantoinsäurealkylester der allgemeinen Formel II umgesetzt werden, Isolierung des Allantoinsäurealkylesters der allgemeinen Formel II und Umsetzung des isolierten Allantoinsäurealkylesters mit einer Arylverbindung der allgemeinen Formel III in konzentrierter, anorganischer Säure bei Temperaturen von Raumtemperatur bis 150 °C mit oder ohne Phasenvermittler, ist ebenfalls Gegenstand der Erfindung.

Auf die beschriebene Art und Weise werden 5-Arylhydantoine in kurzen Reaktionszeiten, guter Reinheit und befriedigenden Ausbeuten hergestellt. Das Verfahren stellt daher eine Bereicherung der Technik dar.

### Beispiel 1

A) Eine Mischung aus 240 g Glyoxylsäuremethylestermethylhemiacetal (2,0 Mol), 400 g Harnstoff (6,66 Mol), 240 g Methanol und 100 g 8%iger, methanolischer Schwefelsäure wurde 5 Stunden unter Rühren auf Rückfluß erhitzt. Danach war die Reaktion beendet und die Reaktionsmischung wurde auf Raumtemperatur abgekühlt, wobei ein farbloser Niederschlag ausfiel, der abfiltriert und nach Waschen mit Methanol bei 70 °C im Vakuum getrocknet wurde.
   Dabei wurden 334,1 g, das sind 88 % der Theorie, Allantoinsäuremethylester erhalten.
   ¹H-NMR (DMSO): δ = 3,63 (s, 3H COOCH₃), 5,32 (t, 1H, J = 8 Hz, CH) 5,82 (s 4H, NH₂), 6,92 (d 2H, J = 8 Hz, NH)
B) 125,1 g Allantoinsäuremethylester (0,66 Mol), 187,5 g Phenol (1,99 Mol) und 225 ml HCl 36 % wurden 4 Stunden auf 83 °C erhitzt. Aus der zu Beginn klaren, farblosen Lösung begann nach ca. 15 bis 20 Minuten ein Niederschlag auszukristallisieren. Nach Beendigung der Reaktion wurde die Reaktionsmischung auf 15 °C gekühlt und weitere 2 Stunden gerührt. Der Niederschlag wurde abfiltriert, mit warmem Wasser gewaschen und im feuchten Zustand in 375 ml Wasser aufgeschlämmt und 2 Stunden lang unter Rückfluß gerührt. Nach Abkühlen, Filtrieren und Waschen mit Wasser wurde der Niederschlag bei 80 °C im Vakuum getrocknet.
   Dabei wurden 79,7 g, das sind 63 % der Theorie, D,L-5-(4-Hydroxyphenyl)-hydantoin mit einer Reinheit von 99,4 % und einem Schmelzpunkt von 267°C erhalten.
   ¹H-NMR (DMSO): δ = 5,06 (s, 1H, CH), 6,83 (d, 2H, J = 8,4 Hz, Ar), 7,16 (d, 2H, J = 8,4 Hz, Ar), 8,33 (s, 1H, NH), 9,6-10,6 (s br, NH)
   Der Anteil des isomeren 5-(2-Hydroxyphenyl)-hydantoins betrug 0,2%.
   Bezogen auf Glyoxylsäuremethylestermethylhemiacetal betrug die Ausbeute über beide Reaktionsstufen A) und B) 55 % der Theorie.
   Die Isomerenreinheit der hergestellten Verbindung wurde mit Hilfe der Hochdruckflüssigkeitschromatographie bestimmt:
   Datensystem: Hewlett-Packard Chemstation
   Säule: Phenyl Bondapak 10 um, Fa. Waters
   Temperatur: Raumtemperatur
   Mobile Phase: Wasser - Acetonitril 88 : 12
   Flußrate: 0,6 ml/min
   Detektor: UV, 254 nm
   Unter diesen Bedingungen betrugen die Retentionszeiten für 5-(4-Hydroxyphenyl)-hydantoin ca. 9 Min. und jene für das isomere 5-(2-Hydroxyphenyl)-hydantoin ca. 11 Min.

### Beispiel 2

Eine Mischung aus 23,8 g Allantoinsäuremethylester (125 mMol), 50 g Benzol (0,64 Mol) und 50 g konzentrierter Schwefelsäure wurde zwei Stunden auf 80 °C erhitzt. Danach war die Reaktion beendet. Die Reaktionsmischung wurde auf Raumtemperatur gekühlt, mit 50%iger Natronlauge auf pH 4 gestellt und mit Essigsäureethylester extrahiert, die organische Phase getrocknet und abgedampft. Der Rückstand wurde aus Wasser umkristallisiert.

Dabei wurden 10,5 g, das sind 47,7 % der Theorie, D,L-5-Phenylhydantoin mit einem Schmelzpunkt von 151 °C erhalten.

¹H-NMR (DMSO): δ = 5,21 (s, 1H, CH), 7,35-7,50 (m, 5H, Ar), 8,46 (s, 1H, NH), 10,6-11,2 (s br, NH)

### Beispiel 3

Eine Mischung aus 83;4 g Allantoinsäuremethylester (0,44 Mol), 146,45 g Brenzkatechin (1,33 Mol) und 250 ml HCl 36 % wurde unter kräftigem Rühren für 2,5 Stunden auf 80 °C erhitzt. Danach war die Reaktion beendet. Die klare Lösung wurde abgekühlt, wobei keine Kristallisation erfolgte. Die Reaktionslösung wurde durch Zugabe von 50%iger Natronlauge auf pH 4,4 gestellt und eingedampft, der verbleibende Rückstand mit Essigsäureethylester im Soxhlet-Extraktor erschöpfend extrahiert, die Essigsäureethylesterphase eingeengt und das Produkt kristallisiert.

Dabei wurden 61,8 g, das sind 67 % der Theorie, D,L-5-(3,4-Dihydroxyphenyl)hydantoin mit einem Schmelzpunkt von 231 bis 233 °C erhalten.

### Beispiel 4

Eine Mischung aus 23,8 g Allantoinsäuremethylester (0,125 Mol), 36,4 g N,N-Dimethylanilin (0,3 Mol) und 12,5 g Essigsäure wurden innerhalb von 30 Minuten mit 50 g konzentrierter Schwefelsäure versetzt, wobei eine Erwärmung des Reaktionsgemisches auf 90 °C erfolgte. Das Reaktionsgemisch wurde 5 Stunden auf 90 bis 95 °C erhitzt, worauf die Reaktion beendet war. Nach Abkühlen der Reaktionsmischung wurde der pH-Wert mit Hilfe von 90 ml 30%iger NaOH auf pH 8 gestellt, worauf Wasser und überschüssiges N,N-Dimethylanilin im Vakuum abdestilliert wurden. Der Rückstand wurde im Soxhlet-Extraktor zunächst mit Ethylacetat, dann mit Methylisobutylketon erschöpfend extrahiert. Das aus dem Methylisobutylketon-Extrakt gewonnene Material wurde säulenchromatographisch gereinigt (Kieselgel, Chloroform : Methanol = 49 : 1). Nach Digerieren in Aceton und Hexan und Umkristallisieren aus Methylisobutylketon wurde D,L-5-(4-Dimethylaminophenyl)hydantoin in Form roter Kristalle mit einem Schmelzpunkt von 228 bis 230 °C erhalten.

¹H-NMR (DMSO): δ = 2,92 (s, 6H, N-CH₃), 5,02 (s, 1H, CH), 6,76 (d, 2H, J = 8,5 Hz, Ar), 7,15 (d, 2H, J = 8,5 Hz, Ar), 8,28 (s, 1H, NH), 10,4-11,0 (s br, NH)

### Beispiel 5

Eine Mischung aus 23,8 g Allantoinsäuremethylester (0,125 Mol) und 50,0 g 1,2,3-Trimethoxybenzol (0,297 Mol) wurden unter kräftigem Rühren mit 50,0 g H₂SO₄ konz. versetzt, wobei sich das Gemisch auf 40 °C erwärmt. Das Reaktionsgemisch wurde 5 Stunden auf 95 bis 98 °C erhitzt und nach dem Abkühlen mit 30%iger NaOH auf pH 8 gestellt. Überschüssiges Trimethoxybenzol wurde mit Hilfe von Hexan extrahiert. Die wässrige Phase wurde im Vakuum eingedampft und der verbleibende Rückstand mit Aceton erschöpfend extrahiert. Das aus dem Aceton-Extrakt isolierte Material wurde über Kieselgel filtriert und säulenchromatographisch gereinigt (Kieselgel, Chloroform : Methanol = 49: 1). Nach Digerieren in Aceton und Hexan und Umkristallisieren aus Methylisobutylketon wurde D,L-5-(3,4,5-Trimethoxyphenyl)hydantoin in Form farbloser Kristalle mit einem Schmelzpunkt von 153,5 bis 154,5 °C erhalten.

¹H-NMR (DMSO): δ = 3,79 (s, 3H, OCH3), 3,82 (s 3H, OCH3), 3,84 (s, J = 3 Hz, OCH3), 5,11 (s, 1H, CH), 6,84 (d, 2H, J = 8,6 Hz, Ar), 6,99 (d, 2H, J = 8,6 Hz, Ar), 8,10 (s, 1H, NH), 10,4-11,0 (s br, NH)

### Beispiel 6

A) Eine Mischung aus 22,2 g Glyoxylsäureethylesterethylhemiacetal (0,15 Mol), 30,0 g Harnstoff (0,5 Mol), 90 g Ethanol und 7,5 g 8%iger ethanolischer Schwefelsäure wurde vier Stunden lang unter Rückfluß erhitzt. Danach war die Reaktion beendet und die Reaktionsmischung wurde auf Raumtemperatur abgekühlt, wobei ein farbloser Niederschlag ausfiel, der abfiltiert, dreimal mit Ethanol gewaschen und im Vakuum bei 70 °C getrocknet wurde. Dabei wurden 24,5 g, das sind 80 % der Theorie, Allantoinsäureethylester erhalten.
   ¹H-NMR (DMSO): δ = 1,19 (t, 3H, J = 7 Hz, COOCH₂CH₃), 4,10 (q, 2H, J = 7Hz, COOCH₂CH₃), 5,29 (t, 1H, J = 8Hz, CH), 5,85 (s, 4H, NH₂), 6,94 (d, 2H, J = 8 Hz, NH)
B) 23,0 g Allantoinsäureethylester (0,11 Mol), 31,2 g Phenol (0,33 Mol) und 37,5 ml HCl 36 % wurden 4 Stunden auf 84 °C erhitzt. Aus der zu Beginn klaren, farblosen Lösung begann nach ca. 15 bis 20 Minuten ein Niederschlag auszukristallisieren.
   Nach Beendigung der Reaktion wurde die Reaktionsmischung innerhalb von einer Stunde auf 15 °C gekühlt und weitere 2 Stunden gerührt. Der Niederschlag wurde abfiltriert, mit zweimal je 75 ml 60 °C heilßem Wasser gewaschen, im feuchten Zustand in 63 ml Wasser aufgeschlämmt und 2 Stunden lang unter Rückfluß gerührt. Nach Abkühlen, Filtrieren, Waschen mit Wasser und Trocknen wurden 13,6 g, das sind 57 % der Theorie D,L-5-(4-Hydroxyphenyl)hydantoin mit einer Reinheit von 99,3 % und einem Schmelzpunkt von 267 °C erhalten. Der Anteil des isomeren 5-(2-Hydroxyphenyl)-hydantoins betrug weniger als 0,1 %.

Bezogen auf Glyoxylsäureethylesterethylhemiacetal betrug die Ausbeute über beide Reaktionsstufen A) und B) 46% der Theorie.

### Beispiel 7

A) Eine Mischung aus 26,4 g Glyoxylsäurepropylesterpropylhemiacetal (0,15 Mol), 30,0 g Harnstoff (0,5 Mol), 600 g Propanol und 7,5 g 8%iger propanolischer Schwefelsäure wurde vier Stunden auf Rückfluß erhitzt. Danach war die Reaktion beendet und die Reaktionsmischung wurde auf Raumtemperatur abgekühlt, wobei ein farbloser Niederschlag ausfiel, der abfiltiert, mit Methanol gewaschen und im Vakuum bei 50 °C getrocknet wurde. Dabei wurden 13,6 g, das sind 42 % der Theorie, kristalliner Allantoinsäurepropylester erhalten.
   ¹H-NMR (DMSO): δ = 0,91 (t, 3H, J = 7,3 Hz, CH₂-CH₂-CH₃), 1,90 (m, 2H, CH₂-CH₂-CH₃), 4,02 (t, 2H, J = 6,4 Hz, O-CH₂-CH₂-CH₃), 5,31 (t, 1H, J = 8 Hz, CH), 5,81 (s, 4H, NH₂), 6,90 (d, 2H, J = 8 Hz, NH)
B) 12,0 g Allantoinsäurepropylester (0,06 Mol), 15,6 g Phenol (0,17 Mol) und 18,8 ml HCl 36 % wurden auf 85 bis 90 °C erhitzt. Aus der zu Beginn klaren, farblosen Lösung begann nach ca. 5 Minuten ein Niederschlag auszufallen. Nach 4 Stunden war die Reaktion beendet. Die Reaktionsmischung wurde auf 15 °C gekühlt und weitere 2 Stunden gerührt. Der Niederschlag wurde abfiltiert, mit zweimal je 75 ml 60 °C heißem Wasser gewaschen, im feuchten Zustand in 32 ml Wasser aufgeschlämmt und 2 Stunden unter Rückfluß gerührt. Nach Abkühlen, Filtrieren und Waschen mit Wasser wurde der Niederschlag bei 70 °C im Vakuum getrocknet. Dabei wurden 5,1 g, das sind 44 % der Theorie, D,L-5-(4-Hydroxyphenyl)hydantoin mit einem Schmelzpunkt von 267 °C und einer Reinheit von mehr als 97 % erhalten. Der Anteil des isomeren D,L-5-(2-Hydroxyphenyl)hydantoins betrug etwa 0,2 %.

### Beispiel 8

A) Eine Mischung aus 30,6 g Glyoxylsäurebutylesterbutylhemiacetal (0,15 Mol), 30,0 g Harnstoff (0,5 Mol), 300 g n-Butanol und 7,5 g 8%iger butanolischer Schwefelsäure wurde insgesamt vier Stunden auf 85 °C erhitzt. Nach Beendigung der Reaktion wurde die entstandene Suspension auf Raumtemperatur gekühlt, wobei ein farbloser Niederschlag ausfiel, der abfiltriert, mit Methanol gewaschen und im Vakuum bei 75 °C getrocknet wurde. Dabei wurden 23,1 g, das sind 66 % der Theorie, kristalliner Allantoinsäurebutylester erhalten.
   ¹H-NMR (DMSO): δ = 0,89 (t, 3H, J = 7,3 Hz, CH₂-CH₂-CH₂-CH₃), 1,34 (m, 2H, CH₂-CH₂-CH₂-CH₃), 1,54 (m, 2H, CH₂-CH₂-CH₂-CH₃), 4,05 (t, 2H, J = 6,4 Hz, O-CH₂-CH₂-CH₂-CH₃)
B) 25,5 g Allantoinsäurebutylester (0,12 Mol), 31,2 g Phenol (0,33 Mol) und 37,5 ml HCl 36 % wurden auf 85 bis 90 °C erhitzt. Aus der zu Beginn klaren, farblosen Lösung begann nach etwa 45 Minuten ein Niederschlag auszukristallisieren. Nach 4 Stunden war die Reaktion beendet. Die Reaktionsmischung wurde auf 15 °C gekühlt, der Niederschlag abfiltiert, mit heißem Wasser gewaschen und im feuchten Zustand in 63 ml Wasser aufgeschlämmt. Die Aufschlämmung wurde 2 Stunden auf Rückfluß erhitzt, die entstandene Suspension auf 30 °C gekühlt, abfiltriert, mit kaltem Wasser nachgewaschen und bei 70 °C im Vakuum getrocknet. Dabei wurden 14,6 g, das sind 53 % der Theorie, D,L-5-(4-Hydroxyphenyl)hydantoin mit einer Reinheit von fast 99 % erhalten. Der Anteil des isomeren 5-(2-Hydroxyphenyl)hydantoins betrug etwa 0,2 %.

## Patentansprüche

1. Verfahren zur Herstellung von 5-Arylhydantoinen der allgemeinen Formel 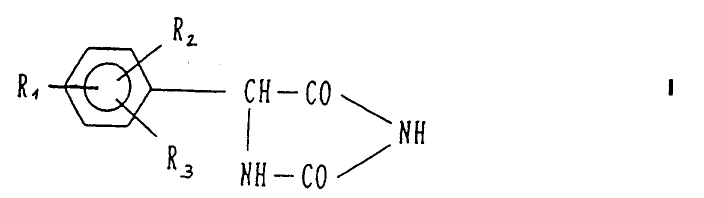 in der R₁, R₂ und R₃ unabhängig voneinander Wasserstoff-, Alkyl- oder Alkoxygruppen mit je 1 bis 6 C-Atomen, Hydroxy-, Halogen- oder durch Acylgruppen mitt 1 bis 8 C-Atomen, durch Alkylgruppen mit 1 bis 6 C-Atomen oder durch Phenylgruppen substituierte Aminogruppen bedeuten, das dadurch gekennzeichnet ist, daß ein Allantoinsäurealkylester der allgemeinen Formel 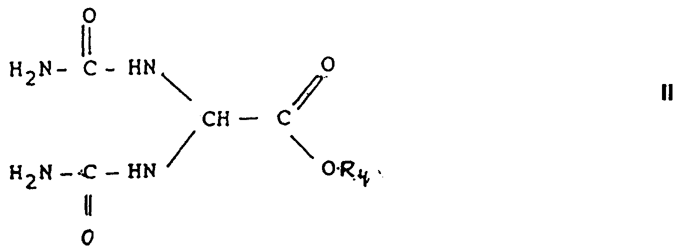 in der R₄ eine Alkylgruppe mit 1 bis 8 C-Atomen bedeutet, in konzentrierter, anorganischer Säure bei Temperaturen von Raumtemperatur bis 150 °C mit oder ohne einen Phasenvermittler mit einer Arylverbindung der allgemeinen Formel 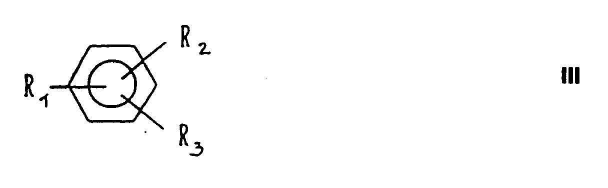 in der R₁, R₂ und R₃ die oben angegebene Bedeutung haben, umgesetzt wird.

2. Verfahren nach Anspruch 1, dadurch gekennzeichnet, daß ein Allantoinsäurealkylester der allgemeinen Formel II nach Anspruch 1 durch Umsetzung von Glyoxylsäurealkylesteralkylhemiacetal der allgemeinen Formel 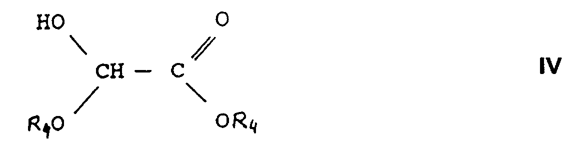 in der R₄ die in Anspruch 1 angegebene Bedeutung hat, mit Harnstoff in einem Alkohol der allgemeinen Formel R₄OH in der R₄ die obgenannte Bedeutung hat, in Gegenwart katalytischer Mengen anorganischer Säure hergestellt, isoliert und in das Verfahren nach Anspruch 1 eingesetzt wird.

3. Verfahren nach einem der Anspüche 1 oder 2, dadurch gekennzeichnet, daß eine Verbindung der allgemeinen Formel II oder der allgemeinen Formel IV, in der R₄ eine Alkylgruppe mit 1 bis 6 C-Atomen bedeutet, eingesetzt wird.

4. Verfahren nach einem der Ansprüche 1 bis 3, dadurch gekennzeichnet, daß als anorganische Säure Salzsäure oder Schwefelsäure eingesetzt wird.

5. Verfahren nach einem der Ansprüche 1 bis 4, dadurch gekennzeichnet, daß die Umsetzung einer Verbindung der allgemeinen Formel II mit einer Verbindung der allgemeinen Formel III bei Temperaturen von 60 bis 120 °C erfolgt.

6. Verfahren nach einem der Ansprüche 1 bis 5, dadurch gekennzeichnet, daß als Arylverbindung der allgemeinen Formel III eine Phenylgruppe, die durch eine oder mehrere Hydroxy-, Alkoxy- oder durch Alkylgruppen substituierte Aminogruppen substituiert ist, eingesetzt wird.

7. Verfahren nach einem der Ansprüche 1 bis 6, dadurch gekennzeichnet, daß als Phasenvermittler Essigsäure eingesetzt wird.
